# EUROPEAN PATENT APPLICATION

(11) **EP 1 688 155 A1**
(43) Date of publication of application: **09.08.2006**
(21) Application number: 04819437.7
(22) Date of filing: 26.11.2004
(51) Int. Cl.: A61L 31/00, A61L 27/00

(54) **CELL GROWTH-INHIBITING FILM, MEDICAL INSTRUMENT AND STENT FOR DIGESTIVE ORGANS**

(30) Priority: 28.11.2003 JP 2003399195; 28.11.2003 JP 2003399197
(71) Applicant: Zeon Medical Inc., Tokyo 1050011 (JP)
(72) Inventor: TANAKA, Masaru, Sapporo-shi, Hokkaido 0010023 (JP); SHIMOMURA, Masatsugu, 1-9-1, Atsubetsu kita 1jyo, Sapporo-shi, Hokkaido 0040071 (JP); TOYOKAWA, Yoshihide, c/o Zeon Medical, Inc., Tokyo 1050011 (JP)
(74) Representative: Albrecht, Thomas
(86) International application number: PCT/JP2004/017572
(87) International publication number: WO 2005/051450

(57) **Abstract**

A cell growth inhibiting film including a resin and having a porous structure formed at least on its surface, a cell growth inhibiting method including causing the surface of a film including a resin and having a porous structure formed at least on its surface to contact cells to inhibit growth of the cells in the contact area, a medical instrument including a medical instrument substrate and a film including a resin and having a porous structure formed at least on its surface, in which the surface of the medical instrument substrate is entirely or partially covered with the film, and a digestive system stent including a stent substrate and a film including a resin and having a porous structure formed by through-holes with an average pore size of 0.1 to 20 µm and a coefficient of variation in pore size of 30% or less, in which the stent substrate is covered with the film. According to the present invention, a material exhibiting cell growth inhibitory effects without using a physiologically active substance and suitable for forming a medical instrument, and a digestive system stent which secures a digestive system tubular cavity in the body and allows a digestive fluid and digestive enzymes contained therein to pass through, but blocks cancer cells, can be provided.

## Description

### TECHNICAL FIELD

The present invention relates to a cell growth inhibiting film, a cell growth inhibiting method using the cell growth inhibiting film, a medical instrument, and a digestive system stent which is placed in a digestive system tubular cavity in the body such as the bile duct, esophagus, duodenum, or large intestine.

### BACKGROUND ART

In regard to the interaction between cells and a material, it is known that the cells are affected not only by the chemical properties of the surface of the material, but also by the minute shape of the surface of the material. For example, JP-A-2002-335949 discloses a film or a stretched film having a honeycomb structure which is obtained by casting a hydrophobic organic solvent solution of a biodegradable and amphiphilic polymer or a polymer mixture of a biodegradable polymer and an amphiphilic polymer onto a substrate, causing the organic solvent to be evaporated and condensed on the surface of the cast organic solvent solution (cast solution), and evaporating the minute waterdrops produced by the condensation. JP-A-2002-335949 describes that this polymer film is useful as a cell culture substrate, since rat fetal cardiac muscle cells cultured on this polymer film were well grown.

JP-A-2003-149096 discloses a blood filter membrane having a honeycomb structure with a specific pore size and pore size variation which is formed by a method similar to that for the film disclosed in JP-A-2002-335949. This filter membrane is used to remove leukocytes from whole blood for transfusion.

In recent years, a medical instrument such as a stent has been placed in the body in order to treat various diseases. For example, when a digestive system tubular cavity in the body, such as the bile duct, esophagus, duodenum, or large intestine, is constricted or obstructed due to cancer cells or the like, a stent has been used as a medical instrument in order to secure the tubular cavity.

When using such a stent, cancer cells grow (infiltrate) with the progress of the cancer, whereby the expanded bile duct or ureter may be constricted or obstructed again. In order to prevent such a problem, JP-T-2001-512354 proposes a medical instrument in which a covering layer is provided on the surface of a medical instrument such as a stent, and a physiologically active substance (e.g. anticancer agent) which can prevent the growth of cancer cells is discharged from the covering layer with time.
However, this medical instrument has a problem in which the physiologically active substance causes significant side effects on the human body to impose a large burden on the patient.

JP-A-2001-327609 or the like discloses a covered stent in which a stent substrate is covered with a resin film. The covered stent is useful for preventing stricture of a tubular cavity in the body due to the growth of cancer cells or the like, since the resin film does not allow the cancer cells to pass through.

However, since the film used for the covered stent cannot allow a digestive fluid such as a pancreatic juice to pass through, the flow of the digestive fluid is hindered due to the covered stent, whereby a serious symptom such as pancreatitis may occur.

### DISCLOSURE OF THE INVENTION

The present invention was achieved in view of the above-described situation of the related art. An object of the present invention is to provide a material exhibiting cell growth inhibitory effects without using a physiologically active substance such as an anticancer agent and suitable for forming a medical instrument, and a digestive system stent which secures a digestive system tubular cavity in the body and allows a digestive fluid and digestive enzymes contained therein to pass through, but blocks cancer cells.

The inventors of the present invention prepared a film having a porous honeycomb structure by casting an organic solvent solution of a resin such as 1,2-polybutadiene onto a substrate using a method similar to the method disclosed in JP-A-2002-335949 and 3P-A-2003-149096. The inventors placed the resulting film in a culture medium and cultured malignant gallbladder carcinoma cells on the film. Surprisingly, the inventors have found that the growth of the cancer cells was remarkably inhibited in contrast to the example using cardiac muscle cells disclosed in JP-A-2002-335949. The inventors also have found that a medical instrument which does not impose a burden on the patient due to side effects caused by a physiologically active substance and can inhibit the progress of cancer can be obtained by covering a medical instrument substrate with the above film.

The inventors have further found that a digestive system stent which secures a digestive system tubular cavity in the body and allows a digestive fluid and digestive enzymes contained therein to pass through, but blocks cancer cells can be obtained by covering a stent substrate with a film including a resin and having a porous structure formed by through-holes with a specific highly controlled pore size. These findings have led to the completion of the present invention.

According to a first aspect of the present invention, there is provided a cell growth inhibiting film comprising a resin and having a porous structure formed at least on its surface.
In the cell growth inhibiting film according to the present invention, the porous structure is preferably a honeycomb structure.
In the cell growth inhibiting film according to the present invention, pores of the porous structure preferably have an average pore size of 0.1 to 100 µm and a coefficient of variation in pore size of 30% or less.

The cell growth inhibiting film according to the present invention is preferably a film or a stretched film obtained by casting a resin organic solvent solution onto a substrate, causing the organic solvent to be evaporated and condensed on the surface of the cast solution, and evaporating minute waterdrops produced by the condensation.

According to a second aspect of the present invention, there is provided a cell growth inhibiting method comprising causing the surface of a film including a resin and having a porous structure formed at least on its surface to contact cells to inhibit growth of the cells in the contact area.
In the cell growth inhibiting method according to the present invention, the porous structure of the film is preferably a honeycomb structure.
In the cell growth inhibiting method according to the present invention, pores of the porous structure of the film preferably have an average pore size of 0.1 to 100 µm and a coefficient of variation in pore size of 30% or less.

In the cell growth inhibiting method according to the present invention, the film is preferably a film or a stretched film obtained by casting a resin organic solvent solution onto a substrate, causing the organic solvent to be evaporated and condensed on the surface of the cast solution, and evaporating minute waterdrops produced by the condensation.

According to a third aspect of the present invention, there is provided a medical instrument comprising a medical instrument substrate and a film including a resin and having a porous structure formed at least on its surface, the surface of the medical instrument substrate being entirely or partially covered with the film.
In the medical instrument according to the present invention, the porous structure of the film with which the medical instrument substrate is covered is preferably a honeycomb structure.
In the medical instrument according to the present invention, pores of the porous structure of the film with which the medical instrument substrate is covered preferably have an average pore size of 0.1 to 100 µm and a coefficient of variation in pore size of 30% or less.

In the medical instrument according to the present invention, the film with which the medical instrument substrate is covered is preferably a film or a stretched film obtained by casting a resin organic solvent solution onto a substrate, causing the organic solvent to be evaporated and condensed on the surface of the cast solution, and evaporating minute waterdrops produced by the condensation.

According to a fourth aspect of the present invention, there is provided a digestive system stent comprising a stent substrate and a film including a resin and having a porous structure formed by through-holes with an average pore size of 0.1 to 20 µm and a coefficient of variation in pore size of 30% or less, the stent substrate being covered with the film.

In the digestive system stent according to the present invention, the porous structure of the film is preferably a honeycomb structure.
In the digestive system stent according to the present invention, the film is preferably a film or a stretched film obtained by casting a resin organic solvent solution onto a substrate, causing the organic solvent to be evaporated and condensed on the surface of the cast organic solvent solution, and evaporating minute waterdrops produced by the condensation.
The digestive system stent according to the present invention is preferably a bile duct stent.

According to the cell growth inhibiting film, the cell growth inhibiting method, and the medical instrument of the present invention, since the cell growth inhibitory effects can be obtained without using a physiologically active substance, side effects due to the physiologically active substance can be prevented.

### BRIEF DESCRIPTION OF THE DRAWINGS

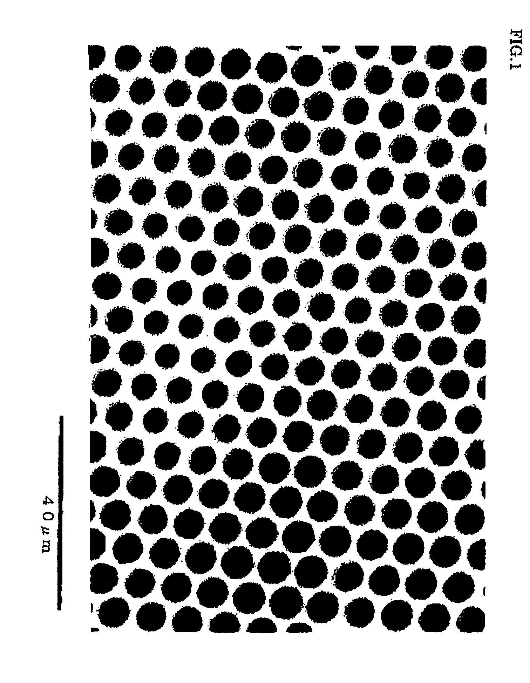

FIG. 1 is a sketch of an optical micrograph of a cell growth inhibiting film having a honeycomb structure according to the present invention.

### BEST MODE FOR CARRYING OUT THE INVENTION

The present invention is described below in detail in the order of 1) a cell growth inhibiting film, 2) a cell growth inhibiting method, 3) a medical instrument, and 4) a digestive system stent.

1) Cell growth inhibiting film
The cell growth inhibiting film according to the present invention includes a resin and has a porous structure formed at least on its surface, and exhibits cell growth inhibitory effects.

The term "cell growth inhibitory effects" used herein refer to the effects of inhibiting the growth of cancer cells or tumor cells and/or the effects of killing cells.
In more detail, when placing the cell growth inhibiting film according to the present invention in a culture medium, disposing strains of cancer cells or tumor cells on the film, and culturing the cells, the growth of the cells is remarkably inhibited or the cells are killed when using the cell growth inhibiting film according to the present invention, although the cells grow normally on a resin film having a flat structure instead of the porous structure.
Therefore, the cell growth inhibiting film according to the present invention is useful as a material for forming a medical instrument or the like.

It suffices that the cell growth inhibiting film according to the present invention have a porous structure at least on its surface. The pores of the porous structure may be through-holes or pores which are not formed through the film.

In the cell growth inhibiting film according to the present invention, it is particularly preferable that the porous structure be a honeycomb structure. The term ''honeycomb structure" used herein refers to a porous structure in which pores with almost the same pore size are regularly arranged. FIG. 1 shows a sketch of an optical micrograph of a film having a honeycomb structure as an example.

It is still more preferable that the cell growth inhibiting film according to the present invention have a continuous porous structure in which the pores of the porous structure are connected in the film.

In the cell growth inhibiting film according to the present invention, the average pore size of the pores of the porous structure is preferably 0.1 to 100 µm, more preferably 0.1 to 20 µm, and still more preferably 0.5 to 10 µm. A film exhibiting more excellent cell growth inhibitory effects can be obtained by forming a porous structure formed by pores having such an average pore size.

The term "pore size" used herein refers to the diameter of the largest inscribed circle for the open shape of the pore. For example, when the open shape of the pore is substantially a circle, the "pore size" refers to the diameter of the circle. When the open shape of the pore is substantially an oval, the "pore size" refers to the minor axis of the oval. When the open shape of the pore is substantially a square, the "pore size" refers to the length of the side of the square. When the open shape of the pore is substantially a rectangle, the "pore size" refers to the length of the short side of the rectangle.
The open shape of each pore of the porous structure is not particularly limited. The open shape of each pore may be arbitrary such as a circle, oval, square, rectangle, or hexagon.

In the cell growth inhibiting film according to the present invention, the coefficient of variation in pore size (= standard deviation / average value × 100 (%)) of the pores of the porous structure is not particularly limited. The coefficient of variation in pore size is preferably 30% or less, and still more preferably 20% or less.
A film exhibiting more excellent cell growth inhibitory effects can be obtained by forming a porous structure formed by pores having such a small coefficient of variation (i.e. having excellent pore size uniformity).

The thickness of the cell growth inhibiting film according to the present invention is not particularly limited. The thickness of the cell growth inhibiting film is usually 0.1 to 100 µm, and preferably 0.5 to 20 µm.

The resin forming the cell growth inhibiting film according to the present invention is not particularly limited. It is preferable that the resin be a polymer compound which is dissolved in an organic solvent and exhibits toxicity to only a small extent.

As examples of the resin forming the cell growth inhibiting film according to the present invention, conjugated diene polymers such as polybutadiene, polyisoprene, styrene-butadiene copolymer, and acrylonitrile-butadiene-styrene copolymer; poly(ε-caprolactone); polyurethane; cellulose polymers such as cellulose acetate, celluloid, cellulose nitrate, acetyl cellulose, and cellophane; polyamide polymers such as polyamide 6, polyamide 66, polyamide 610, polyamide 612, polyamide 12, and polyamide 46; fluorine-containing polymers such as polytetrafluoroethylene, polytrifluoroethylene, and perfluoroethylene-propylene copolymer: styrene polymers such as polystyrene, styrene-ethylene-propylene copolymer, styrene-ethylene-butylene copolymer, styrene-isoprene copolymer, chlorinated polyethylene-acrylonitrile-styrene copolymer, methacrylate-styrene copolymer, styrene-acrylonitrile copolymer, styrene-maleic anhydride copolymer, and acrylate-acrylonitrile-styrene copolymer; olefin polymers such as polyethylene, chlorinated polyethylene, ethylene-α-olefin copolymer, ethylene-vinyl acetate copolymer, ethylene-vinyl chloride copolymer, ethylene-vinyl acetate copolymer, polypropylene, olefin-vinyl alcohol copolymer, and polymethylpentene; formaldehyde polymers such as a phenol resin, amino resin, urea resin, melamine resin, and benzoguanamine resin; polyester polymers such as polybuthylene terephthalate, polyethylene terephthalate, and polyethylene naphthalate; epoxy resin; (meth)acrylic polymers such as poly(meth)acrylate, poly-2-hydroxyethyl acrylate, and methacrylate-vinyl acetate copolymer; norbornene resin; silicone resin; hydroxycarboxylic acid polymers such as polylactic acid, polyhydroxybutyric acid, and polyglycolic acid; and the like can be given.
These resins may be used either individually or in combination of two or more.

A non-biodegradable resin or a biodegradable resin may be used as the resin forming the cell growth inhibiting film according to the present invention. In order to maintain the cell growth inhibitory effects for a long time *in vivo,* it is preferable that the cell growth inhibiting film according to the present invention include a non-biodegradable resin which is not easily decomposed *in vivo.*
It is particularly preferable to use the conjugated diene polymer, styrene polymer, or polyurethane, since a cell growth inhibiting film exhibiting excellent cell growth inhibitory effects can be obtained.

An amphiphilic substance may be added to the resin forming the cell growth inhibiting film according to the present invention.
As examples of the amphiphilic substance added to the resin, a polyethylene glycol-polypropylene glycol block copolymer; an amphiphilic resin having an acrylamide polymer as the main chain skeleton and containing a dodecyl group as a hydrophobic side chain and a lactose group or a carboxyl group as a hydrophilic side chain; an ion complex of an anionic polymer (e.g. heparin, dextran sulfate, or nucleic acid (DNA or RNA)) and a long-chain alkyl ammonium salt; an amphiphilic resin containing a water-soluble protein such as gelatin, collagen, or albumin as a hydrophilic group; amphiphilic resins such as a polylactic acid-polyethylene glycol block copolymer, poly(ε-caprolactone)-polyethylene glycol block copolymer, and polymalic acid-polyalkyl malate block copolymer; and the like can be given.

Since the cell growth inhibiting film according to the present invention exhibits the cell growth inhibitory effects without adding a physiologically active substance, it is preferable not to add a physiologically active substance exhibiting cell growth inhibitory effects from the viewpoint of preventing side effects. Note that a physiologically active substance exhibiting cell growth inhibitory effects may be added in order to obtain higher cell growth inhibitory effects. In this case, since sufficient cell growth inhibitory effects can be obtained by adding only a small amount of physiologically active substance, side effects due to the physiologically active substance can be reduced.

A method of forming the cell growth inhibiting film according to the present invention is not particularly limited. For example, a method may be used which includes casting a resin organic solvent solution onto a substrate, causing the organic solvent to be evaporated and condensed on the surface of the cast solution, and evaporating the minute waterdrops produced by condensation.

A specific method includes (1) a method which includes casting a resin organic solvent solution onto a substrate, causing the organic solvent to be gradually evaporated and condensed on the surface of the cast solution by spraying high-humidity air, and evaporating the minute waterdrops produced by condensation, or (2) a method which includes casting a resin organic solvent solution onto a substrate in air at a relative humidity of 50 to 95%, causing the organic solvent to be evaporated and condensed on the surface of the cast solution, and evaporating the minute waterdrops produced by condensation. According to the above method, a cell growth inhibiting film having a porous honeycomb structure formed by pores with a desired pore size and excellent pore size uniformity can be relatively easily obtained.

The above method is characterized by using the waterdrop produced by condensation as a mold. A film having a continuous porous structure can be obtained by using the waterdrop as a mold.

When forming the cell growth inhibiting film according to the present invention using the above method, since it is necessary to form minute waterdrop particles on the surface of the cast solution, it is preferable to use a water-insoluble organic solvent.

As examples of the organic solvent, halogenated hydrocarbon solvents such as chloroform and methylene chloride; saturated hydrocarbon solvents such as n-pentane, n-hexane, and n-heptane; alicyclic hydrocarbon solvents such as cyclopentane and cyclohexane; aromatic hydrocarbon solvents such as benzene, toluene, and xylene; ester solvents such as ethyl acetate and butyl acetate; ketone solvents such as diethyl ketone and methyl isobutyl ketone; carbon disulfide; and the like can be given.
These solvents may be used either individually or in combination of two or more.

The resin is dissolved in the organic solvent to a concentration of preferably 0.01 to 10 wt%, and still more preferably 0.05 to 5 wt%. If the resin concentration is less than 0.01 wt%, the resulting film may exhibit insufficient mechanical strength. If the resin concentration is 10 wt% or more, a desired porous structure may not be obtained.

When forming a film having a porous structure using the above method, it is preferable to add the above-mentioned amphiphilic substance to the resin. In particular, it is preferable to add an amphiphilic resin (hereinafter called "Cap resin") shown by the following formula which exhibits low water solubility and can be dissolved in an organic solvent.

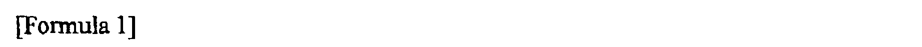

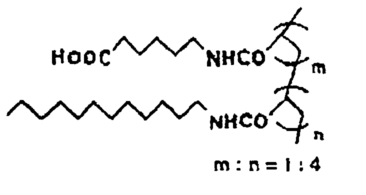

wherein m and n individually represent arbitrary positive integers.
Since the waterdrops are prevented from being fused and are stabilized by adding such an amphiphilic substance, a film having a porous structure with improved pore size uniformity can be obtained. The amphiphilic substance is preferably added so that the weight ratio of the amount of the resin to the amount of the amphiphilic substance is 99:1 to 50:50.

As examples of the substrate onto which the resin organic solvent solution is cast, inorganic substrates such as a glass substrate, metal substrate, and silicon substrate; organic substrates made of polymers such as polypropylene, polyethylene, and polyether ketone; liquid substrates made of liquids such as water, liquid paraffin, and liquid polyether; and the like can be given.

The pore size may be controlled by supplying the cast solution to a supporting layer such as a petri dish while adjusting the resin concentration and the amount of the cast solution, and controlling the temperature and/or the humidity of the atmosphere or air sprayed and the flow rate of air sprayed, or controlling the evaporation rate and/or the condensation rate of the solvent.

It suffices that the high-humidity air sprayed onto the cast solution have a humidity which allows moisture in air to be condensed on the surface of the cast solution. It is preferable that the high-humidity air have a relative humidity of 20 to 100%, and preferably 30 to 80%. An inert gas such as nitrogen or argon may be used instead of air.

The flow rate of the high-humidity air sprayed onto the cast solution is not particularly limited insofar as moisture in air can be condensed on the surface of the cast solution and the solvent used for casting can be evaporated. For example, when forming a film on a glass petri dish with a diameter of 10 cm, the flow rate of the high-humidity air is preferably 1 to 5 l/min.

The high-humidity air is sprayed until a film is formed due to evaporation of the solvent used for casting. The high-humidity air is usually sprayed for 1 to 60 minutes.
The temperature of the atmosphere when spraying the high-humidity air is not particularly limited insofar as the solvent used for casting can be evaporated. The temperature of the atmosphere is preferably 5 to 80°C.

In the present invention, the resulting film having a porous structure may be directly used, or may be stretched to form a stretched film.

The method of stretching the film is not particularly limited. For example, the film having a porous structure may be held on two or more sides and pulled in the stretching direction. The stretching operation may be uniaxial stretching, biaxial stretching, or triaxial stretching. In the present invention, the stretch ratio in the stretching direction is preferably 1.1 to 10, although the stretch ratio is not particularly limited.

In the present invention, the cell growth inhibiting film may be stretched by covering a medical instrument substrate with the cell growth inhibiting film and expanding the medical instrument substrate, as described later. Specifically, a stretched cell growth inhibiting film is obtained by expanding a medical instrument substrate covered with the cell growth inhibiting film according to the present invention.

2) Cell growth inhibiting method
The cell growth inhibiting method according to the present invention includes causing the surface of a film including a resin and having a porous structure formed at least on its surface to contact cells to inhibit growth of the cells in the contact area.
As the film caused to contact the cells, the above-described cell growth inhibiting film is preferably used.

3) Medical instrument
The medical instrument according to the present invention includes a medical instrument substrate and a film including a resin and having a porous structure formed at least on its surface, the surface of the medical instrument substrate being entirely or partially covered with the film.

The term "medical instrument substrate" used herein refers to a substrate which may be used as the medical instrument when covered with the film, but also includes a substrate which may be used as the medical instrument without being covered with the film.
As the film used to cover the medical instrument substrate, the above-described cell growth inhibiting film is preferably used.

Since the medical instrument according to the present invention is covered with the film exhibiting cell growth inhibitory effects on cancer cells or tumor cells, the progress of cancer can be hindered in the contact area of the film. Since the film exhibits the cell growth inhibitory effects without using a physiologicahy active substance such as an anticancer agent, side effects due to the physiologically active substance can be prevented.

As examples of the medical instrument according to the present invention, a stent, catheter, medical tube, and the like can be given. The medical instrument according to the present invention is preferably a stent, and particularly preferably a stent which is placed in a tubular cavity in the body constricted or obstructed due to cancer cells or tumor cells. As examples of such a stent, a ureteral stent, bile duct stent, airway stent, esophagus stent, and large intestine stent, and the like can be given. In particular, a digestive system stent which is placed in a digestive system tubular cavity in the body, such as the bile duct, esophagus, duodenum, or large intestine, is preferable.

The method of covering the medical instrument substrate with the film is not particularly limited. It is preferable to form the film in the same manner as in the method of forming the cell growth inhibiting film according to the present invention, and then cover the medical instrument substrate with the resulting film. In this case, adhesion can be obtained between the film and the medical instrument substrate by merely having the film contact the surface of the medical instrument substrate. Note that means such as an adhesive, fusion using a solvent, or fusion using heat may be arbitrarily used.

As another method of covering the medical instrument substrate with the film, a method of forming the film on the medical instrument substrate using the above-described method can be given.

4) Digestive system stent
The digestive system stent according to the present invention includes a stent substrate and a film including a resin and having a porous structure formed by through-holes with an average pore size of 0.1 to 20 µm and a coefficient of variation in pore size of 30% or less, the stent substrate being covered with the film.

The film used to cover the stent substrate is the above-described cell growth inhibiting film and has a specific structure. In more detail, the film used to cover the stent substrate is a film including a resin and having a porous structure formed by through-holes with an average pore size of 0.1 to 20 µm, and preferably 0.5 to 10 µm, and a coefficient of variation in pore size of 30% or less, and preferably 20% or less. It is preferable that the porous structure of the film be the above-described honeycomb structure.

In general, digestive enzymes have a size of 1×10⁻⁴ to 1×10⁻³ µm, and cancer cells (tumor cells) have a size of about 20 to several hundred microns. On the other hand, the film used in the present invention is a film including a resin and having a porous structure formed by through-holes exhibiting excellent pore size uniformity with an average pore size of 0.1 to 20 µm and a coefficient of variation in pore size of 30% or less, as described above. Therefore, the film allows digestive enzymes to pass through, but blocks cancer cells (tumor cells). If the average pore size of the porous structure is less than 0.1 µm, a digestive fluid and digestive enzymes may not pass through the film. If the average pore size exceeds 20 µm, cancer cells (tumor cells) may pass through the film. If the coefficient of variation in pore size of the pores of the porous structure exceeds 30%, cancer cells may pass through the film even if the average pore size is in the specific range.

It is preferable that the porous structure be a continuous porous structure in which the adjacent pores are connected in the film. This structure reduces the flow resistance when a digestive fluid passes through the film, whereby the digestive fluid can pass through the film at a low pressure in comparison with a film having a porous structure in which the adjacent pores are not connected. Moreover, a digestive fluid secreted at a low pressure such as a pancreatic juice can efficiently pass through the film.
As the method of forming the film used in the present invention, a method similar to the method of forming the cell growth inhibiting film according to the present invention is preferable.

The stent substrate used in the present invention is a substrate which may be used as a stent when covered with the film, but may be a substrate which can be used as a stent without being covered with the film.

The shape of the stent substrate is not particularly limited insofar as the stent substrate is tubular. The stent substrate is usually a tubular body in which linear or strip-shaped materials are connected in a network to form the circumferential wall.

When forming the stent substrate using the linear materials, the diameter of the linear material is preferably 0.05 to 1 mm. When forming the stent substrate using the strip-shaped materials, the strip-shaped material preferably has a width of 0.1 to 10 mm and a thickness of 0.05 to 5 mm.

The size of the tubular body used as the stent substrate varies depending on the size of the tubular cavity in the body in which the stent is placed. The tubular body used as the stent substrate usually has an outer diameter of 2 to 30 mm, an inner diameter of 1 to 29 mm, and a length of 5 to 200 mm. In particular, when the tubular body is used to form a bile duct stent, the tubular body preferably has an outer diameter of 5 to 20 mm, an inner diameter of 4 to 19 mm, and a length of 10 to 100 mm.

As the material for the stent substrate, a synthetic resin or a metal is used. As the synthetic resin, a synthetic resin exhibiting a certain hardness and elasticity is used. The synthetic resin is preferably a biocompatible resin. As specific examples of such a synthetic resin, a polyolefin, polyester, fluororesin, and the like can be given. As examples of the polyolefin, polyethylene and polypropylene can be given. As examples of the polyester, polyethylene terephthalate and polybuthylene terephthalate can be given. As examples of the fluororesin, polytetrafluoroethylene (PTFE), an ethylene-tetrafluoroethylene copolymer (ETFE), and the like can be given. As the metal, a superelastic alloy such as a nickel-titanium (Ti-Ni) alloy, stainless steel, tantalum, titanium, a cobalt-chromium alloy, or the like may be used. Of these, the superelastic alloy is preferably used.

It is particularly preferable to use a Ti-Ni alloy containing 49 to 53 atom% of Ni. The mechanical characteristics of the superelastic alloy may be arbitrarily changed by replacing some of the atoms of the Ti-Ni alloy with another atom in an amount of 0.01 to 10.0% to prepare a Ti-Ni-X alloy (X=Co, Fe, Mn, Cr, V, Al, Nb, W, B, or the like), or replacing some of the atoms of the Ti-Ni-X alloy with another atom in an amount of 0.01 to 30.0% to prepare a Ti-Ni-X alloy (X=Cu, Pb, or Zr), and selecting the cold reduction ratio and/or the final heat treatment conditions.

The stent substrate may be formed by processing the material in the shape of a pipe by laser processing (e.g. YAG laser), electric discharge processing, chemical etching, cutting, or the like.

It is preferable to provide an X-ray marker to the stent substrate so that the position of the stent substrate can be determined by X-ray fluoroscopy when placed in a tubular cavity in the body. The X-ray marker is formed of an X-ray contrast material (X-ray blocking material). This allows the position of the stent substrate to be determined by X-ray fluoroscopy.

As the X-ray blocking material, an X-ray contrast metal such as gold, platinum, a platinum-iridium alloy, platinum, silver, stainless steel, or an alloy of these metals may be suitably used. The X-ray marker may be a resin molded product containing an X-ray contrast substance powder. As the X-ray contrast substance powder, barium sulfate, bismuth subcarbonate, tungsten powder, powder of the above-mentioned metal, or the like may be used.

The digestive system stent according to the present invention is characterized in that the stent substrate is covered with the above-described film. In the digestive system stent according to the present invention, it suffices that at least part of the stent substrate be covered with the above-described film. The stent substrate may be covered with the film on either or both the outer surface and the inner surface of the circumferential wall of the stent substrate.

Since the digestive system stent according to the present invention is formed by covering the stent substrate with the above-described film, the digestive system stent exhibits cell growth inhibitory effects in the contact area of the film. Moreover, the circumferential wall of the stent allows a digestive fluid and digestive enzymes contained therein to pass through, but blocks cancer cells (tumor cells). Therefore, when the digestive system stent according to the present invention is placed in a digestive system tubular cavity in the body, the tubular cavity in the body can be prevented from being constricted due to the growth of cancer cells through the circumferential wall of the digestive stent, and the flow of the digestive fluid and the digestive enzymes is not hindered.

The method of covering the stent substrate with the above-described film is not particularly limited. The film may be merely wound around the stent substrate, or means such as an adhesive, fusion using a solvent, or fusion using heat may be arbitrarily used.

The digestive system stent according to the present invention may be placed in a digestive system tubular cavity in the body in the same manner as a known stent. For example, when the stent substrate is formed of an elastic material such as the superelastic alloy, the stent is inserted into a delivery catheter in a state in which the circumferential wall of the stent is contracted, the delivery catheter is delivered to the target location, and the stent is removed from the delivery catheter to expand the circumferential wall of the stent. When the stent substrate is formed of a material which lacks elasticity such as stainless steel, the stent is provided to enclose a balloon of a balloon catheter, the balloon catheter is delivered to the target location, and the balloon is expanded to expand the circumferential wall of the stent, for example. The stent substrate is usually expanded when placing the stent in the digestive system tubular cavity in the body. Note that the film with which the stent substrate is covered may be stretched by utilizing the expansion of the stent substrate.

The digestive system stent according to the present invention may be placed in an arbitrary digestive system tubular cavity in the body such as the bile duct, esophagus, duodenum, or large intestine. It is preferable to use the digestive system stent according to the present invention as a bile duct stent of which the circumferential wall usually reaches the pancreatic juice outlet when placed in the bile duct.

By using the digestive system stent according to the present invention as a bile duct stent, circulation of the pancreatic juice and digestive enzymes contained therein such as trypsin and lipase is not hindered even if the stent reaches the pancreatic juice outlet when placed in the bile duct, whereby the onset of pancreatitis or the like can be prevented.

### EXAMPLES

The present invention is described below in more detail by way of examples and comparative examples. Note that the present invention is not limited to the following examples.
The cell strains used and their culturing conditions were as follows.

1) Cell strains
Cell strain A: human gallbladder carcinoma cell line NOZ (cell number: JCRB1033)
Cell strain B: human malignant gallbladder carcinoma cell line OCUG-1 (cell number: JCRB0191)
These cell strains were purchased from the Health Science Research Resources Bank of the Japan Health Sciences Foundation.

2) Culturing conditions
The cell strain A (NOZ) was cultured at 37°C and 5% CO₂ in a William's E medium containing fetal bovine serum (10% FBS) and 2 mML-sodium glutamate.
The cell strain B (OCUG-1) was cultured at 37°C and 5% CO₂ in a Dulbecco's modified Eagle's medium containing 10% FBS and 0.5 mM pyruvic acid.

About 1×10⁴ cells per well were plated on a 24-well plate (Falcon 3047) (overnight incubation) to achieve a confluence of about 80% on the following day.

The Dulbecco's modified Eagle's medium was purchased from Invitrogen Corporation. The William's E medium, L-sodium glutamate, and pyruvic acid were purchased from ICN Biomedicals Inc. The fetal bovine serum was purchased from JRH Bioscience.

(Preparation of film used for cell growth inhibition effect evaluation test)
(Example 1)
6 ml of a solution (resin concentration: 0.27 wt%) prepared by dissolving poly(ε-caprolactone) (manufactured by Wako Pure Chemicals Co., Ltd., viscosity average molecular weight: 70,000) and a Cap resin (weight average molecular weight: 62,000, number average molecular weight: 21,000) in chloroform at a weight ratio of 10:1 was uniformly spread on a glass petri dish with a diameter of 10 cm.
Then, high-humidity air with a relative humidity of 70% was sprayed onto the liquid surface on the glass petri dish for one minute at a flow rate of 2 l/min in an atmosphere at a temperature of 23.0°C and a relative humidity of 40% to obtain a film A with a thickness of 1 to 2 µm. The film A was observed using an optical microscope ("BH2" manufactured by Olympus Corporation) at a magnification of 100. It was confirmed that a porous honeycomb structure was formed in the film A. The pores of the porous structure had an average pore size of 3.5 µm and a coefficient of variation in pore size of 9%. The average pore size and the coefficient of variation in pore size were determined by measuring the pore size of all the pores positioned in the microscope field (100×100 µm).

(Examples 2 and 3)
In Examples 2 and 3, films B and C having a porous honeycomb structure were obtained in the same manner as in Example 1 except for changing the temperature of the atmosphere to 24.0°C and 25.0°C, respectively.
Table 1 shows the thicknesses of the films B and C and the average pore size and the coefficient of variation in pore size of the pores of the porous structure.

(Examples 4 to 6)
Films D, E, and F were respectively obtained in the same manner as in Examples 1, 2, and 3 except for using 1,2-polybutadiene ("RB820" manufactured by JSR Corporation) as the resin instead of poly(ε-caprolactone).
As a result of observation using an optical microscope, it was confirmed that a porous honeycomb structure was formed in the films D to F. Table 1 shows the thicknesses of the films D to F and the average pore size and the coefficient of variation in pore size of the pores of the porous structure.

(Examples 7 and 8)
Films G and H were respectively obtained in the same manner as in Examples 1 and 2 except for using polyurethane ("Miractran E385" manufactured by Japan Miractran Co., Ltd.) as the resin instead of poly(ε-caprolactone).
As a result of observation using an optical microscope, it was confirmed that a porous honeycomb structure was formed in the films G and H. Table 1 shows the thicknesses of the films G and H and the average pore size and the coefficient of variation in pore size of the pores of the porous structure.

(Comparative Examples 1 to 3)
The chloroform solution of poly(ε-caprolactone)/Cap resin used in Example 1, the chloroform solution of 1,2-polybutadiene/Cap resin used in Examples 4 to 6, and the chloroform solution of polyurethane/Cap resin used in Examples 7 and 8 were respectively spread on a glass petri dish with a diameter of 10 cm in an amount of 6 ml. Each solution was allowed to stand in an atmosphere at a temperature of 23.0°C and a relative humidity of 40% to evaporate chloroform without spraying high-humidity air to obtain films I to K of Comparative Examples 1 to 3. As a result of observation using an optical microscope, it was found that the films I to K of Comparative Examples 1 to 3 had a flat structure instead of the porous structure. Table 1 shows the thicknesses of the films I to K of Comparative Examples 1 to 3.

**[Table 1]**

| | Resin | Film | Thickness (µm) | Average pore size (µm) | Coefficient of variation in pore size (%) |
|---|---|---|---|---|---|
| Example 1 | Poly(ε-caprolactone) | A | 1 to 2 | 3.5 | 9 |
| Example 2 | Poly(ε-caprolactone) | B | 2 to 3 | 6.4 | 11 |
| Example 3 | Poly(ε-caprolactone) | C | 3 to 4 | 9.1 | 15 |
| Example 4 | 1,2-Polybutadiene | D | 3 to 4 | 3.6 | 7 |
| Example 5 | 1,2-Polybutadiene | E | 4 to 5 | 6.3 | 9 |
| Example 6 | 1,2-Polybuladiene | F | 8 to 10 | 9.4 | 9 |
| Example 7 | Polyurethane | G | 3 to 4 | 4.1 | 25 |
| Example 8 | Polyurethane | H | 6 to 7 | 8.1 | 26 |
| Comparative Example I | Poly(ε-caprolactone) | I | 1 to 2 | - | - |
| Comparative Example 2 | 1,2-Polybutadiene | J | 2 to 3 | - | - |
| Comparative Example 3 | Polyurethane | K | 3 to 4 | - | - |

(Cell growth inhibition effect evaluation test)
Each of the films A to K of Examples 1 to 8 and Comparative Examples 1 to 3 was placed in the William's E medium and the Dulbecco's modified Eagle's medium. The cell strains A and B were disposed on each film and cultured under the above culturing conditions.

(Evaluation of cell growth inhibitory activity)
The cells cultured for a specific number of days were washed twice with a Dulbecco's phosphoric acid buffer (manufactured by Dainippon Sumitomo Pharma Co., Ltd.) which did not contain magnesium, and stained with a Wright solution (blood staining solution manufactured by Wako Pure Chemical Industries, Ltd.) for 10 minutes.
Each stained cell was observed using a phase-contrast microscope (field of view: 100×100 µm). A case where the cell contact area was less than 30% of the field of view was evaluated as "Excellent", a case where the cell contact area was 30% or more and less than 50% of the field of view was evaluated as "Good", and a case where the cell contact area was more than 50% of the field of view was evaluated as "Bad". The evaluation results are shown in Table 2.

**[Table 2]**

| | Film | Cell growth inhibitory activity | |
|---|---|---|---|
| | | Cell strain A | Cell strain B |
| Example 1 | A | Excellent | Excellent |
| Example 2 | B | Good | Good |
| Example 3 | C | Good | Good |
| Example 4 | D | Excellent | Excellent |
| Example 5 | E | Excellent | Excellent |
| Example 6 | F | Good | Good |
| Example 7 | G | Good | Good |
| Example 8 | H | Good | Good |
| Comparative Example I | I | Bad | Bad |
| Comparative Example 2 | J | Bad | Bad |
| Comparative Example 3 | K | Bad | Bad |

As shown in Table 2, it was confirmed that the cell growth inhibiting film according to the present invention exhibits an excellent cell growth inhibitory activity for the cell strain A: human gallbladder carcinoma cell line (NOZ) and the cell strain B: human malignant gallbladder carcinoma cell line (OCUG-1). As a result of comparison between Examples 1 to 3 and Examples 4 to 6, it was confirmed that the cell growth inhibitory activity was increased as the average pore size was decreased.
On the other hand, the cell growth inhibitory activity was not observed when using the films of Comparative Examples 1 to 3 which did not have a porous structure.

(Preparation of film used for digestive enzyme/cell permeation test)
(Example 9)
6 ml of a solution (resin concentration: 0.27 wt%) prepared by dissolving 1,2-polybutadiene ("RB820" manufactured by JSR Corporation) and a Cap resin containing the repeating unit shown by the Formula 1 (weight average molecular weight: 62,000, number average molecular weight: 21,000) in chloroform at a weight ratio of 10:1 was uniformly spread on a glass petri dish with a diameter of 10 cm. Then, high-humidity air with a relative humidity of 70% was sprayed onto the liquid surface on the glass petri dish for one minute at a flow rate of 2 l/min in an atmosphere at a temperature of 23.0°C and a relative humidity of 40% to obtain a film L with a thickness of 3 to 4 µm.

The film L was observed using an optical microscope ("BH2" manufactured by Olympus Corporation) at a magnification of 100. It was confirmed that a porous honeycomb structure formed by through-holes was formed in the film L. The through-holes of the porous structure had an average pore size of 3.6 µm and a coefficient of variation in pore size of 7%.

(Examples 10 and 11)
In Examples 10 and 11, films M and N having a porous honeycomb structure formed by through-holes were obtained in the same manner as in Example 9 except for changing the temperature of the atmosphere to 24.0°C and 25.0°C, respectively. Table 3 shows the thicknesses of the films M and N and the average pore size and the coefficient of variation in pore size of the through-holes of the porous structure.

(Examples 12 to 14)
Films O, P, and Q were respectively obtained in the same manner as in Examples 9 to 11 except for using polyurethane ("Miractran E385" manufactured by Japan Miractran Co., Ltd.) as the resin instead of 1,2-polybutadiene. As a result of observation using an optical microscope, it was confirmed that a porous honeycomb structure was formed in the films O to Q. Table 3 shows the thicknesses of the films O to Q and the average pore size and the coefficient of variation in pore size of the through-holes of the porous structure.

(Comparative Examples 4 and 5)
The chloroform solution of 1,2-polybutadiene/Cap resin used in Example 9 and the chloroform solution of polyurethane/Cap resin used in Example 12 were respectively spread on a glass petri dish with a diameter of 10 cm in an amount of 6 ml. Each solution was allowed to stand in an atmosphere at a temperature of 23.0°C and a relative humidity of 40% to evaporate chloroform without spraying high-humidity air to obtain films R and S of Comparative Examples 4 and 5. As a result of observation using an optical microscope, it was found that the films R and S had a flat structure instead of the porous structure. Table 3 shows the thicknesses of the films R and S.

(Reference Example 1)
A film T of Reference Example 1 was obtained in the same manner as in Example 9 using the chloroform solution of 1,2-polybutadiene/Cap resin used in Example 9 except for spraying high-humidity air with a relative humidity of 70% onto the liquid surface on the glass petri dish for one minute at a flow rate of 5 l/min in an atmosphere at a temperature of 28.0°C instead of spraying high-humidity air with a relative humidity of 70% onto the liquid surface on the glass petri dish for one minute at a flow rate of 21/min in an atmosphere at a temperature of 23.0°C . Table 3 shows the thickness of the film T and the average pore size and the coefficient of variation in pore size of the through-holes of the porous structure.

(Reference Example 2)
A film U of Reference Example 2 was obtained in the same manner as in Example 12 except for uniformly spreading 10 ml of the solution (resin concentration: 0.27 wt%) used in Examples 12 to 14 which was prepared by dissolving the polyurethane resin and the Cap resin in chloroform at a weight ratio of 10:1 1 on a glass petri dish with a diameter of 10 cm instead of uniformly spreading 6 ml of the solution (resin concentration: 0.27 wt%) prepared by dissolving the polyurethane resin and the Cap resin in chloroform at a weight ratio of 10:1 1 on a glass petri dish with a diameter of 10 cm.
Table 3 shows the thickness of the film U and the average pore size and the coefficient of variation in pore size of the through-holes of the porous structure.

**[Table 3]**

| | Resin | Film | Thickness (µm) | Average pore size (µm) | Coefficient of variation in pore size (%) |
|---|---|---|---|---|---|
| Example 9 | 1,2-Polybutadiene | L | 3 to 4 | 3.6 | 7 |
| Example 10 | 1,2-Polybutadiene | M | 4 to 5 | 6.3 | 9 |
| Example 11 | 1,2-Polybutadiene | N | 8 to 10 | 9.4 | 9 |
| Example 12 | polyurethane | O | 3 to 4 | 4.1 | 25 |
| Example 13 | Polyurethane | P | 6 to 7 | 8.1 | 26 |
| Example 14 | Polyurethane | Q | 10 to 12 | 12.4 | 27 |
| Comparative Example 4 | 1,2-Polybutadiene | R | 2 to 3 | - | - |
| Comparative Example 5 | Polyurethane | S | 3 to 4 | - | - |
| Reference Example I | 1,2-Polybutadiene | T | 1 to 2 | 27.5 | 20 |
| Reference Example 2 | Polyurethane | U | 15 to 30 | 18.5 | 38 |

(Digestive enzyme/cell permeation test)
1) Preparation of test solution
   (1) Digestive enzyme test solution
      A trypsin powder was added to a phosphate buffered saline solution (PBS) to prepare 25 g/l of a trypsin PBS solution. This solution was used a trypsin test solution. Likewise, 25 g/l of a lipase PBS solution was prepared using a lipase powder. This solution was used as a lipase test solution.

(2) Cancer cell test solution
The cell strain A (NOZ) was cultured under the above culturing conditions. The resulting NOZ was added to PBS to prepare a cell suspension containing the NOZ at a concentration of 1×10⁶ cell/ml. This cell suspension was used as an NOZ test solution.

The cell strain B (OCUG-1) was cultured under the above culturing conditions. The resulting OCUG-1 was added to PBS to prepare a cell suspension containing the OCUG-1 at a concentration of 1 ×10⁶ cell/ml. This cell suspension was used as an OCUG-1 test solution.

2) Permeation test
Each of the films L to U prepared in Examples 9 to 14, Comparative Examples 4 and 5, and Reference Examples 1 and 2 was placed in a filter holder with a diameter of 10 mm. Each of the test solutions was added dropwise to the film from the upper side at a rate of 0.5 ml/min. After 10 minutes of addition, the solution which passed through the film was collected to obtain 10 ml of a permeated solution. A permeated solution could not obtained when using the films R and S, since the test solutions did not pass through the films.

3) Measurement of amount of digestive enzyme
The amount of digestive enzyme in the solution was measured as follows using a UV-visible spectrophotometer (JASCO V-530).
Each of the trypsin and lipase test solutions (concentration: 25 g/l) before being allowed to passed through the film was diluted with PBS 100 times to a concentration of 0.25 g/l. This concentration was taken as a conversion concentration "0.01Co". Likewise, trypsin and lipase solutions with a conversion concentration of 0.009Co, 0.007Co, 0.005Co, or 0.004Co were prepared. The absorbance (trypsin: 278 nm, lipase: 274 nm) of these solutions was measured to create a conversion concentration-absorbance calibration curve. The absorbance of the trypsin solution was 0.23 Abs and the absorbance of the lipase solution was 0.14 Abs at a concentration of 0.01 Co.

The trypsin test solution and the lipase test solution which had passed through the films L to Q, T, and U were diluted with PBS 100 times, and the absorbance of these solutions was measured. The resulting absorbance was converted into the conversion concentration using the calibration curve to determine the permeation rate (permeated solution concentration / test solution concentration × 100 (%)). The results are shown in Table 4.

**[Table 4]**

| Film | Solution | Absorbance (Abs) | Conversion concentration (Co) | Permeation rate (%) |
|---|---|---|---|---|
| L | Trypsin | 0.23 | 0.01 | 100 |
| | Lipase | 0.14 | 0.01 | 100 |
| M | Trypsin | 0.23 | 0.01 | 100 |
| | Lipase | 0.14 | 0.01 | 100 |
| N | Trypsin | 0.23 | 0.01 | 100 |
| | Lipase | 0.14 | 0.01 | 100 |
| O | Trypsin | 0.23 | 0.01 | 100 |
| | Lipase | 0.14 | 0.01 | 100 |
| P | Trypsin | 0.23 | 0.01 | 100 |
| | Lipase | 0.14 | 0.01 | 100 |
| Q | Trypsin | 0.23 | 0.01 | 100 |
| | Lipase | 0.14 | 0.01 | 100 |
| R | Trypsin | Did not permeate | - | 0 |
| | Lipase | | - | 0 |
| S | Trypsin | Did not permeate | - | 0 |
| | Lipase | | - | 0 |
| T | Trypsin | 0.23 | 0.01 | 100 |
| | Lipase | 0.14 | 0.01 | 100 |
| U | Trypsin | 0.23 | 0.01 | 100 |
| | Lipase | 0.14 | 0.01 | 100 |

As shown in Table 4, it was confirmed that the films L to Q, T, and U allow trypsin and lipase to completely pass through.

(4) Measurement of amount of cancer cell
The cell concentration of the NOZ test solution and the OCUG-1 test solution which had passed through the films L to Q, T, and U was measured using a hemacytometer. The results are shown in Table 5.

**[Table 5]**

| Film | Solution | Cell concentration in permeated solution (cell/ml) |
|---|---|---|
| L | NOZ OCUG-1 | 0 |
| M | NOZ OCUG-1 | 0 |
| N | NOZ OCUG-1 | 0 |
| O | NOZ OCUG-1 | 0 |
| P | NOZ OCUG-1 | 0 |
| Q | NOZ OCUG-1 | 0 |
| R | NOZ OCUG-1 | Did not permeate Did not permeate |
| S | NOZ OCUG-1 | Did not permeate Did not permeate |
| T | NOZ | 0.3×10⁶ |
| | OCUG-1 | 0.2×10⁶ |
| U | NOZ | 0.1×10⁶ |
| | OCUG-1 | 0.1×10⁶ |

As shown in Table 5, the films L to Q did not allow NOZ and OCUG-1 to pass through. On the other hand, it was confirmed that the films T and U allow NOZ and OCUG-1 to pass through. Therefore, in order to provide a digestive system stent with the function of allowing digestive enzymes to pass through while blocking cancer cells, it is necessary to cover the stent substrate with a resin film having a porous structure formed by through-holes with an average pore size of 0.1 to 20 µm and a coefficient of variation in pore size of 30% or less.

### INDUSTRIAL APPLICABILITY

The present invention provides (1) the cell growth inhibiting film exhibiting excellent cell growth inhibitory effects without using a physiologically active substance and suitable for forming a medical instrument, (2) the cell growth inhibiting method using the cell growth inhibiting film according to the present invention, (3) the medical instrument in which a medical instrument substrate is covered with the cell growth inhibiting film according to the present invention, and (4) the digestive system stent which secures a digestive system tubular cavity in the body, and allows a digestive fluid and digestive enzymes contained therein to pass through, but blocks cancer cells.

## Claims

1. A cell growth inhibiting film comprising a resin and having a porous structure formed at least on its surface.

2. The cell growth inhibiting film according to claim 1, wherein the porous structure is a honeycomb structure.

3. The cell growth inhibiting film according to claim 1, wherein pores of the porous structure have an average pore size of 0.1 to 100 µm.

4. The cell growth inhibiting film according to claim 1, wherein pores of the porous structure have a coefficient of variation in pore size of 30% or less.

5. The cell growth inhibiting film according to claim 1, which is a film or a stretched film obtained by casting a resin organic solvent solution onto a substrate, causing the organic solvent to be evaporated and condensed on the surface of the cast solution, and evaporating minute waterdrops produced by the condensation.

6. A cell growth inhibiting method comprising causing the surface of a film including a resin and having a porous structure formed at least on its surface to contact cells to inhibit growth of the cells in the contact area.

7. The cell growth inhibiting method according to claim 6, wherein the porous structure of the film is a honeycomb structure.

8. The cell growth inhibiting method according to claim 6, wherein pores of the porous structure of the film have an average pore size of 0.1 to 100 µm.

9. The cell growth inhibiting method according to claim 6, wherein pores of the porous structure of the film have a coefficient of variation in pore size of 30% or less.

10. The cell growth inhibiting method according to claim 6, wherein the film is a film or a stretched film obtained by casting a resin organic solvent solution onto a substrate, causing the organic solvent to be evaporated and condensed on the surface of the cast solution, and evaporating minute waterdrops produced by the condensation.

11. A medical instrument comprising a medical instrument substrate and a film including a resin and having a porous structure formed at least on its surface, the surface of the medical instrument substrate being entirely or partially covered with the film.

12. The medical instrument according to claim 11, wherein the porous structure of the film is a honeycomb structure.

13. The medical instrument according to claim 11, wherein pores of the porous structure of the film have an average pore size of 0.1 to 100 µm.

14. The medical instrument according to claim 11, wherein pores of the porous structure of the film have a coefficient of variation in pore size of 30% or less.

15. The medical instrument according to claim 11, wherein the film is a film or a stretched film obtained by casting a resin organic solvent solution onto a substrate, causing the organic solvent to be evaporated and condensed on the surface of the cast solution, and evaporating minute waterdrops produced by the condensation.

16. A digestive system stent comprising a stent substrate and a film including a resin and having a porous structure formed by through-holes with an average pore size of 0.1 to 20 µm and a coefficient of variation in pore size of 30% or less, the stent substrate being covered with the film.

17. The digestive system stent according to claim 16, wherein the porous structure of the film is a honeycomb structure.

18. The digestive system stent according to claim 16, wherein the film is a film or a stretched film obtained by casting a resin organic solvent solution onto a substrate, causing the organic solvent to be evaporated and condensed on the surface of the cast organic solvent solution, and evaporating minute waterdrops produced by the condensation.

19. The digestive system stent according to claim 16, which is a bile duct stent.
